# EUROPÄISCHE PATENTSCHRIFT

(11) **EP 0 700 922 B1**
(45) Veröffentlichungstag und Bekanntmachung des Hinweises auf die Patenterteilung: **08.03.2000**
(21) Anmeldenummer: 95113287.7
(22) Anmeldetag: 24.08.1995
(51) Int. Cl.: C07F 9/165, C07F 9/02

(54) **Stabilisierte Dithiophosphorsäurepolysulfide**
Stabilised dithiophosphoric acid polysulphides
Polysulfures dithiophosphoriques stabilisés

(30) Priorität: 06.09.1994 DE 4431727
(43) Veröffentlichungstag der Anmeldung: 13.03.1996
(73) Patentinhaber: RHEIN-CHEMIE RHEINAU GmbH, 68219 Mannheim (DE)
(72) Erfinder: Graf, Hans-Joachim, Dr., D-68219 Mannheim (DE); Steger, Lothar, Dr., D-81545 München (DE); Knörr, Klaus, D-67459 Böhl-Iggelheim (DE); Schulz, Hartmut, Dr., D-69121 Heidelberg (DE); Schäfer, Volker, Dr., D-67122 Altrip (DE); Scholl, Thomas, Dr., D-51469 Bergisch Gladbach (DE); Schubart, Rüdiger, Dr., D-51467 Bergisch Gladbach (DE); Schweiger, Manfred, D-68642 Bürstadt (DE)
(74) Vertreter: Feldhues, Michael L.F., Dr.

(56) Entgegenhaltungen:
- DE-A- 2 249 090
- US-A- 4 496 495
- US-A- 5 208 362
- PLAST., RUBBER COMPOS. PROCESS. APPL. (PRPAEP,09598111);93; VOL.20 (3); PP.179-184, RUBBER RES. INST.;PIASTOW; 05-820; POL. (PL) PYSKLO L 'Mechanism of the vulcanization of cis-1,4-polyisoprene with mixtures of bis(diisopropyl)thiophosphoryl disulfide and dithiodimorpholine'
- CHEMICAL ABSTRACTS, vol. 079, no. 10, 10. September 1973, Columbus, Ohio, US; abstract no. 061078, TRDLICKA V ET AL 'Thermal stability of some dithiophosphoric acid derivatives' & CHEM. PRUM. (CHPUA4);73; VOL.23 (5); PP.238-40, VYS. SK. CHEMICKOTECHNOL.;PRAGUE; CZECH.

## Beschreibung

Dithiophosphorsäurepolysulfide sind bekannt, ebenso ihre Anwendung als Vulkanisationsmittel oder Vulkanisationsbeschleuniger für die Kautschukvulkanisation (vgl. DDR-Patentschrift 228 722, DE-OS 22 49 090) Plast; Rubber Compos. Process. Appl.; 93; Vol. 20(3) und EP-A 383 102). Da es schwierig ist, die Richtwerte für Nitrosamine in der Luft bei der Kautschukvulkanisation einzuhalten, besteht besonderes Interesse an Kautschukhilfsmitteln, die vulkanisationsaktiv sind aber keine gefährlichen Zersetzungsprodukte, insbesondere keine Nitrosamine bilden. Hier nehmen die Dithiophosphorsäurepolysulfide eine herausragende Stellung ein.

Dithiophosphorsäurepolysulfide können hergestellt werden aus den Natriumsalzen der entsprechenden Dithiophosphorsäuren und Dischwefeldichlorid (S₂Cl₂). Sie stellen grünlich gelbe Öle dar, die einen unangenehmen mercaptanartigen Geruch besitzen und die sich - insbesondere bei Anwesenheit von Wasser - leicht unter Abspaltung von Schwefelwasserstoff zersetzen.

Die Dithiophosphorsäurepolysulfide entsprechen der Formel 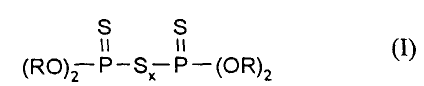 in der
- R: ein C₁-C₂₀-Alkylrest, ein Cycloalkylrest, ein Arylrest oder ein Aralkylrest ist und
- x: eine ganze Zahl von 2 bis 15 bedeutet.

Bevorzugt sind R ein C₄-C₈-Alkylrest
x ist bevorzugt 4 bis 6 und ganz besonders bevorzugt 4.

Gegenstand dieser Erfindung sind durch einen Zusatz von 1 bis 20 Gew.-% einer Monocarbonsäure der Formel R'COOH mit R' = C₁-C₂₀-Alkyl, Cycloalkyl, Aryl oder Aralkyl und 1 bis 20 Gew.-% Calciumoxid oder Zinkoxid, jeweils bezogen auf die Mischung gegen thermische und hydrolytische Zersetzung stabilisierter Dithiophosphorsäurepolysulfide der Formel (I).

Die stabilisierten Produkte sind hellgelbe fast geruchlose Öle, die auch im wäßrigen Medium und in der Wärme keine Zersetzung erleiden.

Die stabilisierenden Carbonsäure und Oxide können den Dithiophosphorsäurepolysulfiden in beliebiger Weise zugefügt werden. Bevorzugt setzt man sie unmittelbar nach der Herstellung zu und bevorzugt wendet man sowohl Carbonsäuren als auch Metalloxide an. Dabei ist es wieder bevorzugt zuerst die Carbonsäure und dann das Metalloxid zuzusetzen.

Bevorzugte stabilisierte Dithiophosphorsäurepolysulfide sind z.B. Dibutyldithiophosphorsäurepolysulfid, Di-2-ethylhexyldithiophosphorsäurepolysulfide, bevorzugte Carbonsäuren sind z.B. 2-Ethylhexansäure, n-Hexansäure, Kokospalmkernölfettsäure und das bevorzugte Metalloxid ist Zinkoxid. Die stabilisierenden Mengen der Carbonsäuren sind 1 bis 20 %, bevorzugt 1 bis 5 %, bezogen auf die Mischung und die Mengen der Metalloxide ebenfalls 1 bis 20, bevorzugt 1 bis 5 Gew.-%, bezogen auf die Mischung.

Die stabilisierten Dithiophosphorsäurepolysulfide können in der gleichen Weise wie nicht stabilisierte Produkte zur Vulkanisation von Kautschuken eingesetzt werden, ohne irgendwelche Einbußen an Wirksamkeit. Die Vorteile der stabilisierten Produkte liegen in ihrer Unempfindlichkeit gegen Hitze und Wasser, der deutlich geringeren Geruchsbelästigung und vor allem in der zuverlässigeren Dosierbarkeit.

### Beispiel

### Stabilisierung von Dibutyldithiophosphorsäurepolysulfid

200 g Dibutyldithiophosphorsäure werden zusammen mit 60 g Wasser in einem Kolben vorgelegt und langsam unter Rühren mit 124 g Natronlauge (25 %ig) versetzt. Nach 1-stündigem Rühren bei Zimmertemperatur werden 52 g Dischwefeldichlorid langsam zugetropft. Man rührt etwa 2 Stunden nach und trennt die wäßrige Phase von der organischen Phase im Scheidetrichter. Der organischen Phase werden 8 g 2-Ethylhexansäure und danach 8 g Zinkoxid zugefügt. Die erhaltene Mischung wird 2 Stunden lang auf 70°C im Vakuum gehalten und danach über Kieselgur filtriert.
Ausbeute: 220 g, gelbes, wenig riechendes Öl.

## Patentansprüche

1. Stabilisierte Dithiophosphorsäurepolysulfide der Formel (I) 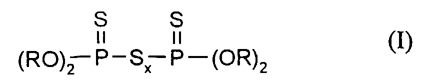 worin
R ein C₁-C₂₀-Alkyl-, ein Cycloalkyl-, ein Aryl- oder ein Aralkylrest und
x eine ganze Zahl von 2 bis 15 ist,
enthaltend als Stabilisator 1 bis 20 Gew.-% einer Carbonsäure der allgemeinen Formel R'COOH, worin R' ein C₁-C₂₀-Alkylrest, ein Cycloalkylrest, ein Arylrest oder ein Aralkylrest ist und 1 bis 20 Gew.-% Calciumoxid oder Zinkoxid.

## Claims

1. Stabilised dithiophosphoric acid polysulfides of the formula (I) 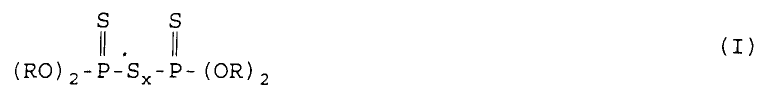 in which
R is a C₁-C₂₀ alkyl, cycloalkyl, aryl or aralkyl residue and
x is an integer from 2 to 15,
containing as stabiliser 1 to 20 wt.% of a carboxylic acid of the general formula R'COOH, in which R' is a C₁-C₂₀ alkyl residue, a cycloalkyl residue, an aryl residue or an aralkyl residue, and 1 to 20 wt.% of calcium oxide or zinc oxide.

## Revendications

1. Polysulfures d'acides dithiophosphoriques stabilisés de formule (I) 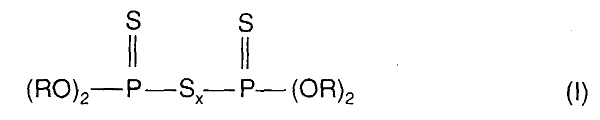 dans laquelle
R représente un groupe alkyle en C₁-C₂₀, cycloalkyle, aryle ou aralkyle et
x est un nombre entier allant de 2 à 5, contenant en tant que stabilisants 1 à 20 % en poids d'un acide carboxylique de formule générale R'COOH dans laquelle R' représente un groupe alkyle en C1-C20, cycloalkyle, aryle ou aralkyle, et 1 à 20 % en poids d'oxyde de calcium ou d'oxyde de zinc.
